# EUROPÄISCHE PATENTSCHRIFT

(11) **EP 1 030 605 B1**
(45) Veröffentlichungstag und Bekanntmachung des Hinweises auf die Patenterteilung: **20.08.2003**
(21) Anmeldenummer: 98966237.4
(22) Anmeldetag: 13.11.1998
(51) Int. Cl.: A61B 17/02, A61B 17/58, A61F 2/44, A61F 2/46

(54) **GERÄTESYSTEM ZUR OPERATIVEN KORREKTUR VON WIRBELVERSCHIEBUNGEN**
OPERATIVE SYSTEM TO CORRECT VERTEBRAL BODY SPLITTING
SYSTEME D'APPAREILS POUR CORRECTION PAR OPERATION DE DEPLACEMENTS DE VERTEBRES

(30) Priorität: 13.11.1997 DE 19750382
(43) Veröffentlichungstag der Anmeldung: 30.08.2000
(73) Patentinhaber: Centerpulse Orthopedics Ltd., 6340 Baar (CH)
(72) Erfinder: BETZ, Augustin, D-78479 Reichenau (DE); KLEIN, Jürgen, D-97990 Weikersheim (DE); OEXLE, Helmut, D-78315 Radolfzell (DE); TODT, Lutz Dr., D-78465 Konstanz-Dettingen (DE)
(74) Vertreter: Manitz, Finsterwald & Partner Gbr
(86) Internationale Anmeldenummer: EP9807281
(87) Internationale Veröffentlichungsnummer: WO99025253

(56) Entgegenhaltungen:
- EP-A- 0 077 159
- EP-A- 0 796 593
- DE-C- 4 416 605
- US-A- 3 486 505
- US-A- 5 431 658
- US-A- 5 599 279

## Beschreibung

Die Erfindung betrifft ein Gerätesystem zur operativen Korrektur von Wirbelverschiebungen und zur Stabilisierung reponierter Wirbelkörper, gemäß dem Oberbegriff des Anspruchs 1.

In der Wirbelsäulenchirurgie ist es bekannt, zur Stabilisierung lumbaler Wirbelsäulensegmente unterschiedliche Implantate zu verwenden und mittels dieser Implantate nach vorhergehender Reposition der Wirbelkörper die Relativlage der Wirbelkörper zu fixieren. Die Aufrichtung des Zwischenwirbelraumes und das Einbringen der erforderlichen Implantate von anterior ist auch bereits durch laparoskopische Operation möglich. Da bei derartigen Operationen nicht nur der durch Destruktion der Bandscheibe verringerte und gegebenenfalls sogar nicht mehr vorhandene Abstand zwischen den Wirbelkörpern wieder hergestellt werden muß, sondern auch relativ zueinander verschobene Wirbelkörper wieder in ihre zumindest angenäherte Sollstellung zurückgeführt werden müssen, ist es erforderlich, den Chirurgen ein Gerätesystem oder Instrumentarium zur Verfügung zu stellen, das zum einen die Durchführung dieser schwierigen Operationen erleichtert und zum anderen sicherstellt, daß die in diesem kritischen Wirbelsäulenbereich bestehende Gefahr der Verletzung von Nervensträngen weitestgehend ausgeschaltet wird. Außerdem muß dieses Gerätesystem zur Durchführung laparoskopischer Operationen geeignet sein, um durch minimal-invasives Zugehen von anterior den großen belastenden

Eingriff für den Patienten zu vermeiden, der bei einer offenen Wirbelsäulenoperation unvermeidbar ist.

Die Merkmale des Oberbegriffs des Anspruchs 1 sind aus der EP-A-0 796 593 bekannt.

Aufgabe der Erfindung ist es daher, ein Gerätesystem der eingangs angegebenen Art zu schaffen, das insbesondere im Rahmen minimal-invasiver Wirbelsäulenoperationen verwendbar ist und eine feinfühlige und sichere Reposition von Wirbelkörpern mit anschließender exakter Positionierung von in einfacher Weise zu fixierenden Implantaten ermöglicht, welche im Knochen der benachbarten Wirbelkörper dauerhaft verankerbar sind und die geforderte Stabilisierung der Wirbelsäulensegmente gewährleisten.

Diese Aufgabe wird nach der Erfindung mit den Merkmalen des Anspruchs 1 gelöst.

Besondere Bedeutung kommt im Rahmen dieses Gerätesystems den Korrektur- und Stützeinheiten zu, die das Kernstück dieses Gerätesystems bilden. Mittels dieser Korrektur- und Stützeinheiten, die nach der Restaurierung der Bandscheibenhöhe, die durch wechselweises Einbringen von Platzhaltern in aufsteigender Größe realisiert wird, in das Bandscheibenfach eingebracht und dabei mit ihrem einen Teil an dem einen Wirbelkörper und mit ihrem anderen Teil an dem anderen Wirbelkörper fixiert werden, wird es ermöglicht, durch relatives Verstellen der beiden Teile die jeweils geforderte Reposition feinfühlig und exakt durchzuführen. In das Bandscheibenfach werden dabei zwei derartige Korrektur- und Stützeinheiten zumindest im wesentlichen mittensymmetrisch eingebracht, wobei die Reposition in einer Mehrzahl von Einzelschritten durchgeführt werden kann und dabei zu jedem Zeitpunkt der Operation eine sichere, den auftretenden Verschiebekräften standhaltende Verankerung mit den Wirbelkörpern einerseits und die Aufrechterhaltung des Wirbelabstandes gewährleistet ist.

Eine bevorzugte Ausführungsform der zur Einbringung in ein Bandscheibenfach bestimmten Korrektur- und Stützeinheit, die auch unabhängig von den weiteren Bestandteilen des Gerätesystems einen eigenständigen Erfindungsgegenstand darstellt, zeichnet sich dadurch aus, daß sie ein oberes und ein unteres Trageteil umfaßt, daß beide Trageteile außenseitig mit Elementen zur Verankerung in den angrenzenden Wirbelkörpern, insbesondere in den Grund- und Deckplatten dieser Wirbelkörper versehen sind, daß die beiden Trageteile zumindest in Richtung ihrer gegenseitigen Verschiebbarkeit formschlüssig zueinander geführt sind, und daß das integrierte Stellorgan aus einer sich in Axialrichtung der Korrektur- und Stützeinheit erstreckenden, in einem Trageteil drehbar und axial fixiert gelagerten und mit dem anderen Trageteil in Gewindeeingriff stehenden Stellschraube besteht.

In spezieller Ausgestaltung ist dabei die Korrektur- und Stützeinheit in Form einer etwa mittig geteilten, zylindrischen Schraube mit betätigungsseitigem zylindrischen Kopfteil ausgebildet, wobei die Stellschraube in einer Hälfte des Kopfteils drehbar gelagert und zur Betätigung an der außenliegenden Stirnfläche des Kopfteils zugänglich ist, und die aufeinander gleitenden Schraubteilhälften relativ zueinander formschlüssig über Koppelelemente geführt sind.

Auf diese Weise wird eine einerseits leichtgängige und feinfühlige Relativverstellung der beiden Schraubteilhälften gewährleistet und andererseits sichergestellt, daß die Richtung der angreifenden Kraft durch die Formschlußverbindung über die Koppelelemente genau vorgegeben ist und kein störendes und gegebenenfalls gefährliches Verdrehen der Korrektur- und Stützeinheit als Gesamtheit auftreten kann.

Die Kupplung zwischen den beiden Schraubteilhälften erfolgt bevorzugt über eine Schwalbenschwanzverbindung, und die beiden Schraubteilhälften weisen außenseitig ein sich bei vorgegebener Relativlage zu einem durchgehenden Gewinde ergänzendes Schneidgewinde auf. Das Schneidgewinde gewährleistet eine auch die Übertragung großer Kräfte ermöglichende sichere Verankerung der Schraubteilhälften in den Grund- und Deckplatten bzw. Knochen der relativ zueinander zu verschiebenden Wirbelkörper und hat den zusätzlichen Vorteil, daß das geschnittene Gewinde nach der nacheinander erfolgenden Entfernung der beiden bei einer Operation verwendeten Korrektur- und Stützeinheiten verwendbar ist für das mit einem korrespondierenden Außengewinde versehene zylindrische Implantat, das in das vorgeschnittene Gewinde eingedreht werden kann. Bevorzugt sind dabei die Flankenhöhen des Schneidgewindes größer und die Flankenwinkel des Schneidgewindes kleiner als die Flankenhöhen bzw. die Flankenwinkel der mit einem Außengewinde gleicher Steigung versehenen Zylinderimplantate, wodurch sich eine Positionierung der Implantate unter gleichzeitiger Erzielung hoher Klemm- und Haltekräfte erreichen läßt.

Das zylindrisch ausgebildete Kopfteil der Korrektur- und Stützeinheiten ist stirnflächenseitig mit Kupplungsausnehmungen und/oder Kupplungsansätzen für ein Betätigungsorgan sowie einer Gewindebohrung zur Verbindung mit dem Betätigungsorgan versehen. Als Betätigungsorgan findet bevorzugt eine Hülsenanordnung mit entsprechendem Drehgriff Verwendung, durch die auch ein mit der Stellschraube kuppelbarer Betätigungsstab geführt ist. Alle Teile sind dabei so dimensioniert und gestaltet, daß sie über Trokare eingeführt und bedient werden können.

Die beiden Schraubgewindehälften besitzen in Axialrichtung unterschiedliche Länge, wobei an das vom Kopfteil abgewandte und entferntere Schraubteilende eine kantenfreie Schutzplatte angeformt sein kann.

Nach einer weiteren Ausgestaltung der Erfindung sind die beiden relativ zueinander verstellbaren Trageteile mit flächigen, insbesondere plattenförmigen Elementen lösbar gekoppelt, die ihrerseits über Vorsprünge, Zähne und dergl. an den Deckplatten der angrenzenden Wirbelkörper in Form von Implantaten fixierbar sind, d.h. diese plattenförmigen Elemente verbleiben nach der Entfernung der Korrektur- und Stützeinheiten im Bandscheibenfach, und die eingebrachten Implantate stützen sich dann an diesen plattenförmigen Elementen ab. Die bevorzugt aus Titan-Hohlschrauben bestehenden Implantate werden mit Spongiosa gefüllt, so daß nach der primären mechanischen Stabilität, die nach einer Operation erreicht wird, die endgültige Stabilisierung nach dem knöchernen Durchbau erfolgen kann, wozu auch die erwähnten plattenförmigen Elemente perforiert ausgebildet sind.

Zu erwähnen ist ferner, daß zwischen den beiden Trageteilen der Korrektur- und Stützeinheit auch eine von der Stirnfläche des Kopfteils her betätigbare Spreizanordnung vorgesehen sein kann, die bei entsprechender Ausgestaltung der gegenseitigen Führung der beiden Trageteile zumindest zum Teil dazu verwendet werden kann, die erforderliche Distraktion des jeweiligen Bandscheibenfachs zu erreichen.

Weitere vorteilhafte Ausgestaltungen der Erfindung sind in den Unteransprüchen angegeben.

Ein Ausführungsbeispiel der Erfindung wird nachfolgend unter Bezugnahme auf die Zeichnung erläutert; in der Zeichnung zeigt:
- Fig. 1: eine perspektivische Seitenansicht einer Korrektur- und Stützeinheit nach der Erfindung,
- Fig. 2: eine schematische Darstellung zur Erläuterung der praktischen Anwendung der Korrektur- und Stützeinheit nach Fig. 1
- Fig. 3: eine Axialschnittdarstellung der Grundelemente der Korrektur- und Stützeinheit nach Fig. 1,
- Fig. 4: eine Stirnansicht des Kopfteils der Anordnung nach Fig.3,
- Fig. 5: eine schematische, teilweise geschnittene Darstellung einer Betätigungs-Hülsenanordnung für die Korrektur- und Stützeinheit,
- Fig. 6: eine schematische Darstellung einer Führungs- und Zentrierhülse mit Platzhalterfunktion, und
- Fig. 7: eine perspektivische Darstellung einer Ausführungsform eines perforierten Hohlzylinderimplantats.

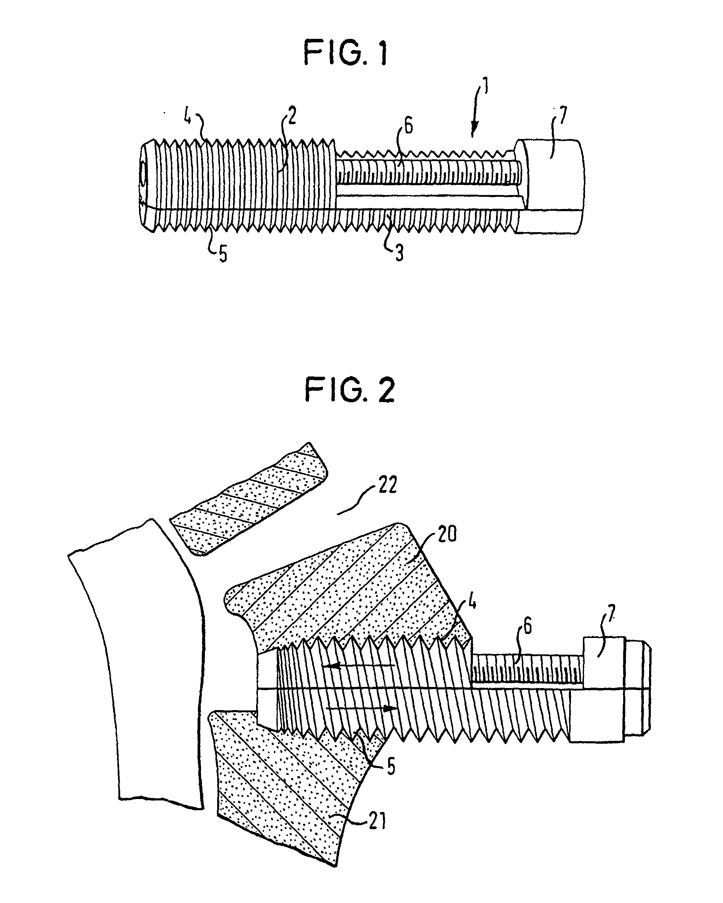

Die Korrektur- und Stützeinheit 1 nach Fig. 1 besteht im wesentlichen aus einer in einer Axialebene getrennten Schraube mit einem oberen Schrauben- bzw. Trageteil 2 und einem unteren Schrauben- bzw. Trageteil 3, wobei das untere Trageteil 3 endseitig mit einem zylindrischen Kopfteil 7 versehen bzw. materialeinheitlich mit diesem ausgebildet ist.

Außenseitig sind die Trageteile 2, 3 mit einem als Schneidegewinde ausgebildeten Schraubgewinde versehen, wobei die als Verankerungselemente dienenden Gewindeflanken 4, 5 bei entsprechender gegenseitiger Ausrichtung der beiden Trageteile 2, 3 ein Gewinde mit durchgehendem Gewindegang bilden.

Die beiden Trageteile 2, 3 sind relativ zueinander über eine formschlüssige Kupplung axial und radial zueinander geführt und sie sind relativ zueinander mittels eines als Schraube ausgebildeten Stellorgans 6 verschiebbar, wobei dieses Stellorgan 6 von der Stirnfläche des Kopfteils her bedienbar ist.

Fig. 2 zeigt schematisch einen Wirbelsäulenabschnitt mit mehreren Wirbelkörpern, wobei die Wirbelkörper 20 und 21 entsprechend einem degenerativen Bandscheibenschaden gegeneinander versetzt dargestellt sind. Diese Wirbelkörper 20, 21 müssen im Rahmen einer insbesondere laparoskopisch durchzuführenden Operation in eine Relativlage zurückgeführt werden, die der Sollposition entspricht oder dieser zumindest angenähert ist, um den betroffenen Patienten von dem mit der Fehlstellung der Wirbelkörper verbundenden Schmerzsyndrom zu befreien.

Bei der grundsätzlich bekannten Operationstechnik muß im Regelfall zunächst eine Distraktion des Bandscheibenfachs erfolgen, wobei dies durch wechselweises Einbringen von Platzhaltern in aufsteigender Größe durchgeführt werden kann und die notwendige Endgröße für die Restaurierung der Bandscheibenhöhe preoperativ bestimmt wird. In das Bandscheibenfach werden dann etwa parallel zueinander und mittensymmetrisch zwei Korrektur- und Stützeinheiten 1, gegebenenfalls nach entsprechenden Vorbohrungen, eingebracht, wobei - wie dies in Fig. 2 zu sehen ist - über die Schneidgewindeflanken 4, 5 eine stabile und sichere Verankerung der oberen und unteren Trageteile im Wirbelkörper bzw. in den entsprechenden Wirbelkörper-Deckplatten erreicht wird. Durch Betätigung der Stellschraube 6 kann - wie in Fig. 2 gezeigt - eine Relativverschiebung der benachbarten und mit den beiden Tragteilen der Korrektur- und Stützeinheit form- und kraftschlüssig gekoppelten Wirbelkörper 20, 21 erfolgen. Der Verschiebevorgang kann dabei äußerst feinfühlig und in den jeweils möglichen und zulässigen Schritten durchgeführt werden, wobei von Bedeutung ist, daß aufgrund der Formschlußführung zwischen den beiden Trageteilen die Verschieberichtung exakt vorgegeben ist und kein Verdrehen der Korrektureinheit auftreten kann. Die form- und kraftschlüssige und damit entsprechend feste Verbindung zwischen den Tragteilen und den Wirbelkörpern erbringt auch den wesentlichen Vorteil, daß jegliche, in diesem kritischen Operationsbereich äußerst gefährliche Abrutscheffekte der Verstellorgane, wie sie bei herkömmlichen Instrumentarien nicht ausgeschlossen werden können, mit Sicherheit vermieden sind.

Wenn die Reposition der Wirbelkörper erreicht ist, kann eine der beiden Korrektur- und Stützeinheiten nach vorheriger Einbringung eines Interims-Platzhalters in Form einer Distanzlaschen aufweisenden Hülse entfernt und ein passendes Hohlzylinderimplantat unter Ausnutzung der bereits vorhandenen Gewindegänge eingebracht werden. Der gleiche Vorgang wird dann mit dem zweiten Korrektur- und Stützelement durchgeführt, so daß nach Abschluß der Operation die wiederhergestellte Relativlage der benachbarten Wirbelkörper 20, 21 durch zwei Hohlzylinderimplantate, insbesondere in Form.von mit entsprechendem Außengewinde versehenen Titan-Hohlschrauben, fixiert ist und die endgültige Stabilität der geschaffenen Verbindung nach dem knöchernen Durchbau durch die mit Spongiosa gefüllte Hohlschraube erreicht werden kann.

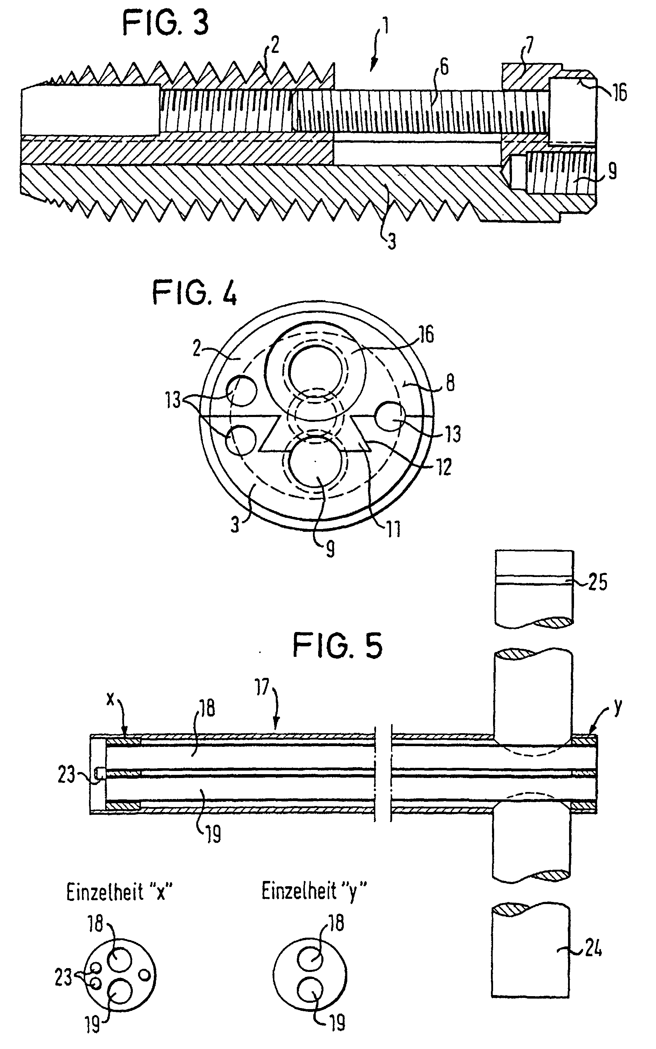

Fig. 3 zeigt eine konstruktive Ausführungsform der Korrektur- und Stützeinheit 1, und die stirnseitige Ansicht nach Fig. 4 läßt erkennen, in welcher Weise die Korrektur- und Stützeinheit 1 mit den zugehörigen Betätigungsorganen gekoppelt und verbunden werden kann.

Das obere Trageteil 2 gleitet flächig auf dem unteren Trageteil 3, wobei die Relativverstellung über ein in Form einer Schraube 6 ausgebildetes Stellorgan 6, das bevorzugt ein Feingewinde besitzt, erfolgen kann. Das Stellorgan 6 ist im Kopfteil in hier nicht näher dargestellter Weise drehbar fixiert, so daß bei einem Drehen des in einer Schraubkopfaufnahme angeordneten Schraubenkopfs die jeweils gewünschte Relativverstellung erfolgt. Das Kopfteil 7 ist des weiteren mit einer Gewindebohrung 9 versehen, über die die Korrektur- und Stützeinheit 1 mit einem hülsenförmigen Betätigungsorgan 17, wie sie in Fig. 5 gezeigt ist, form- und kraftschlüssig verbunden werden kann.

Die Relativführung zwischen dem oberen Trageteil 2 und dem unteren Trageteil 3 erfolgt bevorzugt über eine in Fig. 4 zu sehende Schwalbenschwanzverbindung 11, 12. An der Stirnfläche 8 des Kopfteils 7 sind neben der Ausnehmung 16 und dem Verbindungsgewinde 9 noch gegeneinander versetzte Kupplungsausnehmungen 13 vorgesehen, in die entsprechende Stifte des Betätigungsorgans eingreifen können, so daß definierte Relativlagen zwischen Betätigungsorgan und Korrektur- und Stützeinheit 1 gewährleistet sind und diese Relativlagen auch über entsprechende Markierungen von außen erkennbar sind.

Die in Fig. 5 gezeigte Betätigungshülsenanordnung ist an die Korrekturund Stützeinheit 1 angepaßt und weist innerhalb der Hülse Führungsrohre 18, 19 auf, durch die zum einen eine Betätigung des Stellorgans 6 erfolgen kann und zum anderen eine in das stirnflächenseitige Gewinde 9 eingreifende Fixierschraube eingebracht werden kann. Die mit einem Handgriff 24 versehene Hülse 17 ist an ihren beiden Enden mit scheibenförmigen Einsätzen versehen, wie sie in den Einzelheiten x und y gezeigt sind. Über diese Einsätze sind die Führungsrohre 18, 19 gehaltert, und außerdem trägt der am freien Ende vorgesehene Einsatz Kuppelstifte 23, die in die entsprechenden Ausnehmungen der Korrektur- und Stützeinheit eingreifen.

Der Griff 24 ist um 90° gedreht gezeichnet, und die Kuppelstifte 23 sind in Flucht mit der griffseitig vorgesehenen Markierung 25.

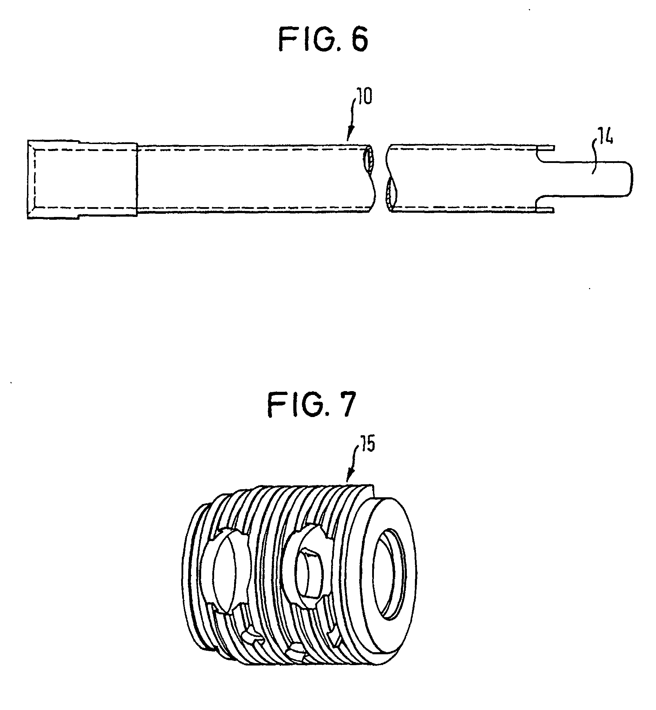

Um jeweils eine der beiden Korrektur- und Stützeinheiten 1 nach erfolgter Reposition der Wirbelkörper ohne Veränderung der durch die Reposition erreichten neuen Relativlage der benachbarten Wirbelkörper entfernen zu können, muß ein Interims-Platzhalter 10, wie er in Fig. 6 gezeigt ist, eingebracht werden, durch den dann die jeweilige Korrektur- und Stützeinheit 1 nach außen entfernt werden kann. Der Hülsenform aufweisende Interims-Platzhalter 10 wird so gestaltet, daß seine beiden endseitig vorgesehenen und einander diametral gegenüberliegenden Distanzlaschen 14, die der Höhe des jeweiligen Bandscheibenfachs angepaßt sind, beidseitig der Korrektur- und Stützeinheit eingeführt werden können. Nach dem Herausschrauben der Korrektur- und Stützeinheit 1 wird der gegenseitige Abstand der Wirbelkörper durch die Distanzlaschen 14 des Platzhalters 10 aufrechterhalten wird. Eine Relativverschiebung der Wirbelkörper wird durch die andere noch eingeschraubte Korrektur- und Stützeinheit verhindert. Ist eine Korrektur- und Stützeinheit entfernt, wird durch die Hülse 10 das passende Hohlzylinderimplantat 15 eingebracht und unter Ausnutzung des über die Korrektur- und Stützeinheit bereits geschnittenen Gewindes zwischen die Wirbelkörper eingeschraubt und dort dauerhaft fixiert. In entsprechender Weise wird dann das zweite Korrektur- und Stützelement durch ein weiteres Hohlzylinderimplantat ersetzt.

Ein Beispiel eines solchen Hohlzylinderimplantats 15 ist in Fig. 7 gezeigt, und es ist dabei zu erkennen, daß solche Implantate vorzugsweise die Form einer perforierten Hohlschraube besitzen, die mit Spongiosa gefüllt werden kann, um den anschließenden knöchernen Durchbau der geschaffenen Verbindung zu gewährleisten.

Für die Erfindung von wesentlicher Bedeutung ist die Korrektur- und Stützeinheit 1. Sämtliche Elemente und Einheiten sind bei der Durchführung laparoskopischer Operationen über die üblichen Trokare handhabbar und problemfrei bedienbar.

## Patentansprüche

1. Gerätesystem zur operativen Korrektur von Wirbelverschiebungen und zur Stabilisierung reponierter Wirbelkörper, mit einem Interims-Platzhalter (10) und mit perforierten Hohlzylinderimplantaten (15), die mit außenliegenden Fixier organen versehen sind,
**gekennzeichnet durch**
zweiteilig ausgebildete, mittels eines integrierten Stellorgans (6) relativ zueinander geführt in Axialrichtung verschiebbare Korrekturund Stützeinheiten (1) sowie den jeweiligen Korrektur- und Stützeinheiten (1) in ihren distanzhaltenden Abmessungen angepaßte Interims-Platzhalter (10), wobei die Hohlzylinderimplantate (15) bei reponierten Wirbelkörpern gegen die Korrektur- und Stützeinheiten (1) austauschbar sind.

2. System nach Anspruch 1, **dadurch gekennzeichnet, daß** die zur Einbringung in ein Bandscheibenfach bestimmte Korrektur- und Stützeinheit (1) ein oberes und ein unteres Trageteil (2, 3) umfaßt, daß beide Trageteile (2, 3) außenseitig mit Elementen (4, 5) zur Verankerung in den angrenzenden Wirbelkörpern, insbesondere in den Grund- und Deckplatten dieser Wirbelkörper versehen sind, daß die beiden Trageteile (2, 3) zumindest in Richtung ihrer gegenseitigen Verschiebbarkeit formschlüssig zueinander geführt sind, und
daß das integrierte Stellorgan (6) aus einer sich in Axialrichtung der Korrektur- und Stützeinheit (1) erstreckenden, in einem Trageteil (3) drehbar und axial fixiert gelagerten und mit dem anderen Trageteil (2) in Gewindeeingriff stehenden Stellschraube besteht.

3. System nach Anspruch 2, **dadurch gekennzeichnet, daß** die Korrektur- und Stützeinheit (1) in Form einer insbesondere mittig geteilten, zylindrischen Schraube mit betätigungsseitigem zylindrischen Kopfteil (7) ausgebildet ist, daß die Stellschraube (6) in einer Hälfte des Kopfteils (7) drehbar gelagert und zur Betätigung an der außenliegenden Stirnfläche (8) des Kopfteils (7) zugänglich ist, und daß die aufeinander gleitenden Schraubteilhälften (2, 3) relativ zueinander formschlüssig über Koppelelemente (11, 12) geführt sind.

4. System nach Anspruch 3, **dadurch gekennzeichnet, daß** die Koppelelemente (11, 12) eine Schwalbenschwanzverbindung bilden.

5. System nach Anspruch 3 oder 4, **dadurch gekennzeichnet, daß** die beiden Schraubteilhälften (2, 3) außenseitig ein sich bei vorgegebener Relativlage zu einem durchgehenden Gewinde ergänzendes Schneidgewinde (4, 5) aufweisen.

6. System nach Anspruch 5, **dadurch gekennzeichnet, daß** der Außendurchmesser des Schneidgewindes (4, 5) zumindest im wesentlichen dem Außendurchmesser des Kopfteils (7) entspricht.

7. System nach einem oder mehreren der vorhergehenden Ansprüche, **dadurch gekennzeichnet, daß** die axiale Länge einer Schraubteilhälfte (2) geringer ist als die axiale Länge der anderen Schraubteilhälfte (3).

8. System nach Anspruch 7, **dadurch gekennzeichnet, daß** die axial kürzere Schraubteilhälfte (2) mit der Stellschraube (6) gekoppelt ist.

9. System nach einem der vorhergehenden Ansprüche, **dadurch gekennzeichnet, daß** die Stellschraube (6) ein Feingewinde geringer Steigung aufweist.

10. System nach einem der vorhergehenden Ansprüche, **dadurch gekennzeichnet, daß** das Kopfteil (7) stirnflächenseitig mit Kupplungsausnehmungen (13) und/oder Kupplungsansätzen für ein Betätigungsorgan aufweist.

11. System nach Anspruch 10, **dadurch gekennzeichnet, daß** im Bereich des Außenumfangs der Stirnfläche (8) mehrere Ausnehmungen (13) für Mitnehmerstifte vorgesehen sind, und daß das Betätigungsorgan mit einer ebenfalls in der Stirnfläche (8) vorgesehenen Gewindebohrung (9) verschraubbar ist.

12. System nach einem der vorhergehenden Ansprüche, **dadurch gekennzeichnet, daß** die Flankenhöhe des Schneidgewindes (4, 5) größer und die Flankenwinkel des Schneidgewindes (4, 5) kleiner sind als die Flankenhöhen bzw. die Flankenwinkel der mit einem Außengewinde gleicher Steigung versehenen Hohlzylinderimplantate (15).

13. System nach einem der vorhergehenden Ansprüche, **dadurch gekennzeichnet, daß** am freien Ende des axial längeren Trageteils (3) mit Außengewinde (5) eine die Bewegung des kürzeren Trageteils (2) begrenzende und innerhalb der Umrißkontur der geteilten Schraube gelegene Schutzscheibe vorgesehen ist.

14. System nach einem oder mehreren der vorhergehenden Ansprüche, **dadurch gekennzeichnet, daß** die beiden relativ zueinander verstellbaren Trageteile (2, 3) mit flächigen, insbesondere plattenförmigen Elementen lösbar gekuppelt sind, die ihrerseits über Vorsprünge, Zähne, Haken und dergl. an den Grund- und Deckplatten der angrenzenden Wirbelkörper in Form von Implantaten fixierbar sind.

15. System nach einem oder mehreren der vorhergehenden Ansprüche, **dadurch gekennzeichnet, daß** zwischen den beiden Trageteilen (2, 3) eine von der Stirnfläche (8) des Kopfteils (7) her betätigbare Spreizanordnung vorgesehen ist.

16. System nach Anspruch 1, **dadurch gekennzeichnet, daß** der Interims-Platzhalter (10) als Zentrierhülsenteil mit endseitig vorgesehenen, einander diametral gegenüberliegenden, stabil ausgeführten Distanzlaschen (14) ausgebildet ist.

17. System nach einem oder mehreren der vorhergehenden Ansprüche, **dadurch gekennzeichnet, daß** alle Elemente des Systems über Trokare einführ- und bedienbar sind.

## Claims

1. Apparatus system for the operative correction of vertebral displacements and for the stabilizing of repositioned vertebrae having an interim spacer (10) and having perforated hollow cylinder implants (15) which are provided with external fixing members,
**characterized by**
correction and support units (1) which are formed in two parts and which are displaceable in the axial direction guided relative to one another by means of an integrated setting member (6), as well as by interim spacers (10) which are matched in their spacing dimensions to the respective correction and support units (1), wherein, with repositioned vertebrae, the hollow cylinder implants (15) are replaceable by the correction and support units (1).

2. System in accordance with claim 1, **characterized in that** the correction and support unit (1) which is intended to be introduced into an intervertebral disc space includes an upper and a lower carrier part (2, 3); **in that** both carrier parts (2, 3) are provided at the outer side with elements (4, 5) for anchoring in the adjacent vertebrae, in particular in the base and cover plates of these vertebrae; **in that** the two carrier parts (2, 3) are guided in a shape-matched manner with respect to one another, at least in the direction of their mutual displaceability; and **in that** the integrated setting member (6) consists of a setting screw which extends in the axial direction of the correction and support unit (1), which is rotationally and axially fixedly mounted in a carrier part (3) and which is in threaded engagement with the other carrier part (2).

3. System in accordance with claim 2, **characterized in that** the correction and support unit (1) is provided in the form of a cylindrical screw, in particular a centrally divided cylindrical screw, with a cylindrical head part (7) at the actuation end; **in that** the setting screw (6) is rotatably mounted in one half of the head part (7) and is accessible at the external end surface (8) of the head part (7) for actuation; and **in that** the screw halves (2, 3) which slide along one another are guided in a shape-matched manner relative to one another via coupling elements (11, 12).

4. System in accordance with claim 3, **characterized in that** the coupling elements (11, 12) form a dovetail connection.

5. System in accordance with claim 3 or claim 4, **characterized in that** the two screw halves (2,3) have at the outer side a cutting thread (4, 5) which is complementary to a through-going thread in a predetermined relative position.

6. System in accordance with claim 5, **characterized in that** the outer diameter of the cutting thread (4, 5) corresponds at least substantially to the outer diameter of the head part (7).

7. System in accordance with one or more of the preceding claims, **characterized in that** the axial length of one screw half (2) is less than the axial length of the other screw half (3)

8. System in accordance with claim 7, **characterized in that** the axially shorter screw half (2) is coupled to the setting screw (6).

9. System in accordance with any one of the preceding claims, **characterized in that** the setting screw (6) has a fine thread of lesser pitch.

10. System in accordance with any one of the preceding claims, **characterized in that** the head part (7) has coupling cut-outs (13) and/or coupling extensions for an actuating member at the end face.

11. System in accordance with claim 10, **characterized in that** a plurality of cut-outs (13) for driver pins are provided in the region of the outer periphery of the end surface (8); and **in that** the actuating member can be screwed with a threaded bore (9) which is likewise provided in the end face (8).

12. System in accordance with any one of the preceding claims, **characterized in that** the flank height of the cutting thread (4, 5) is greater and the flank angles of the cutting thread (4, 5) are smaller than the flank heights and flank angles respectively of the hollow cylinder implants (15), which are provided with an external thread of the same pitch.

13. System in accordance with any one of the preceding claims, **characterized in that** a protective disc which limits the movement of the shorter carrier part (2) and which lies within the outline contour of the divided screw is provided at the free end of the axially longer carrier part (3) with external thread (5).

14. System in accordance with one or more of the preceding claims, **characterized in that** the two carrier parts (2, 3), which are displaceable with respect to one another, are releasably coupled to areal, in particular plate-like elements, which can in turn be fixed via projections, teeth, hooks and the like to the base and cover plates of the adjacent vertebrae in the form of implants.

15. System in accordance with one or more of the preceding claims, **characterized in that** a spreading arrangement, which can be actuated from the direction of the end face (8) of the head part (7), is provided between the two carrier parts (2, 3).

16. System in accordance with claim 1, **characterized in that** the interim spacer (10) is formed as a centering sleeve part with diametrically opposed spacer lugs (14) which are provided at the end sides and which are formed to be stable.

17. System in accordance with one or more of the preceding claims, **characterized in that** all elements of the system can be introduced and handled through trocars.

## Revendications

1. Système d'appareils pour correction par opération de déplacements de vertèbres et pour la stabilisation de corps de vertèbres, ayant été réduits, avec un élément de maintenance d'espace intérimaire (10) et avec un implant à cylindre creux (15) perforé, munis d'organes de fixation extérieurs
**caractérisé par** des unités de correction et de soutien (1) réalisées en deux parties, guidées l'une par rapport à l'autre à l'aide d'un organe de réglage (6) intégré et déplaçables en direction axiale, ainsi que par des éléments de maintenance d'espace intérimaire (10), adaptés, quant à leurs dimensions de maintien d'espace, aux unités de correction et de soutien (1) respectives, les implants à cylindre creux (15), dans le cas où les corps de vertèbres sont réduits, étant remplaçables par les unités de correction et de soutien (1).

2. Système selon la revendication 1, **caractérisé en ce que** l'unité de correction et de soutien (1), déterminée pour être introduite dans un compartiment à disque vertébral, comprend une partie support supérieure et une partie support inférieure (2, 3), **en ce que** les deux parties support (2, 3) sont munies extérieurement d'éléments (4, 5) pour l'ancrage dans les corps de vertèbre limitrophes, en particulier dans les plaques de base et de couvercle de ces corps de vertèbre, **en ce que** les deux parties support (2, 3) sont guidées l'une par rapport à l'autre, avec une liaison par ajustement de forme, dans la direction de leur mobilité réciproque, et
**en ce que** l'organe de réglage (6) intégré est formé d'une vis de réglage, s'étendant dans la direction axiale de l'unité de correction et de soutien (1), fixée, avec possibilité de rotation mais immobilisée axialement, dans une partie support (3) et mise en prise par filetage avec l'autre partie support (2).

3. Système selon la revendication 2, **caractérisé en ce que** l'unité de correction et de soutien (1) est réalisée à la forme d'une vis cylindrique, en particulier divisée centralement, vis comportant une partie tête (7) cylindrique, située côté actionnement, **en ce que** la vis de réglage (6) est montée à rotation dans une moitié de la partie tête (7) et est accessible, pour l'actionnement, sur la face frontale (8) extérieure de la partie tête (7), et **en ce que** les moitiés de parties de vis (2, 3), glissant l'une i sur l'autre, sont guidées l'une par rapport à l'autre avec une liaison à ajustement de forme, par l'intermédiaire d'éléments de couplage (11, 12).

4. Système selon la revendication 3, **caractérisé en ce que** les éléments de couplage (11, 12) forment une liaison à queue d'aronde.

5. Système selon la revendication 3 ou 4, **caractérisé en ce que** les deux moitiés de parties de vis sont présentes extérieurement, un filetage découpeur ou taraudeur (4, 5), qui, dans le cas d'une position relative prédéterminée, se complètent pour former un filetage continu.

6. Système selon la revendication 5, **caractérisé en ce que** le diamètre extérieur du filetage découpeur ou taraudeur (4, 5) correspond au moins sensiblement au diamètre extérieur de la partie tête (7).

7. Système selon l'une ou plusieurs des revendications précédentes, **caractérisé en ce que** la longueur axiale d'une moitié de partie de vis (2) est inférieure à la longueur axiale de l'autre moitié de partie de vis (3).

8. Système selon la revendication 7, **caractérisé en ce que** la moitié de partie de vis (2) axialement plus courte est couplée à la vis de réglage (6).

9. Système selon l'une des revendications précédentes, **caractérisé en ce que** la vis de réglage (6) présente un filetage fin, de faible pas.

10. Système selon l'une des revendications précédentes, **caractérisé en ce que** la partie tête (7) présente, côté face frontale, des évidements d'accouplement (13) et/ou des appendices d'accouplement pour un organe d'actionnement.

11. Système selon la revendication 10, **caractérisé en ce que**, dans la zone de la périphérie extérieure de la face frontale (8), sont prévus plusieurs évidements (13) pour des tiges d'entraînement, et **en ce que** l'organe d'actionnement est susceptible d'être vissé dans un trou taraudé (9), également prévu dans la face frontale (8).

12. Système selon l'une des revendications précédentes, **caractérisé en ce que** la hauteur de flanc du filetage découpeur-taraudeur (4, 5) est supérieure et les angles de flanc du filetage découpeur-taraudeur (4, 5) sont inférieurs aux hauteurs de flanc, respectivement aux angles de flanc, des implants à cylindre creux (15) munis d'un filetage extérieur de pas identique.

13. Système selon l'une des revendications précédentes, **caractérisé en ce que**, à l'extrémité libre de la partie support (13), axialement plus longue, munie d'un filetage extérieur (5), est prévue une rondelle de protection délimitant le déplacement de la partie support la plus courte et placée à l'intérieur du contour de profil de la vis divisée.

14. Système selon l'une ou plusieurs des revendications précédentes, **caractérisé en ce que** les deux parties support (2, 3), déplaçables l'une par rapport à l'autre, sont couplées de façon désolidarisable à des éléments plats, en particulier se présentant sous forme de plaques, qui, de leur côté, sont susceptibles d'être fixées, sous la forme d'implants, par l'intermédiaire de saillies, de dents, de crochets et analogues, sur les plaques de fond et de couvercle des corps de vertèbre limitrophes.

15. Système selon l'une ou plusieurs des revendications précédentes, **caractérisé en ce qu'**un dispositif d'écartement, actionnable depuis la face frontale (8) de la partie tête (7), est prévu entre les deux parties support (2, 3).

16. Système selon la revendication 1, **caractérisé en ce que** l'élément de maintien d'espacement intérimaire (10) est réalisé sous la forme de partie de douille de centrage, avec des pattes d'espacement (14) stables, prévues côté extrémité, diamétralement opposées les unes aux autres.

17. Système selon l'une ou plusieurs des revendications précédentes, **caractérisé en ce que** tous les éléments du système dont susceptible d'être introduits et manoeuvrés par l'intermédiaire d'un trocart.
